# FASCICULE DE BREVET EUROPEEN

(11) **EP 1 052 989 B1**
(45) Date de publication et mention de la délivrance du brevet: **30.07.2003**
(21) Numéro de dépôt: 99901703.1
(22) Date de dépôt: 03.02.1999
(51) Int. Cl.: A61K 31/425, A61P 21/00, A61P 25/14

(54) **APPLICATION DU 2-AMINO-6- TRIFLUOROMETHOXY- BENZOTHIAZOLE POUR LA PREVENTION OU LE TRAITEMENT DES DYSFONCTIONNEMENTS DU CERVELET**
VERWENDUNG VON 2-AMINO-6-TRIFLUOROMETHOXYBENZOTHIAZOL ZUR HERSTELLUNG EINES ARZNEIMITTELS ZUR VORBEUGUNG ODER BEHANDLUNG VON ZEREBELLUMKRANKHEITEN
USE OF 2-AMINO-6- TRIFLUOROMEHTHOXY- BENZOTHIAZOLE FOR PREVENTING OR TREATING CEREBELLUM DYSFUNCTION

(30) Priorité: 06.02.1998 FR 9801402
(43) Date de publication de la demande: 22.11.2000
(73) Titulaire: Aventis Pharma S.A., 92160 Antony (FR)
(72) Inventeur: BOHME, Andrees, F-75020 Paris (FR); BOIREAU, Alain, F-94370 Sucy en Brie (FR); CANTON, Thierry, F-93270 Sevran (FR); IMPERATO, Assunta, F-75014 Paris (FR)
(86) Numéro de dépôt international: FR9900230
(87) Numéro de publication internationale: WO99039710

(56) Documents cités:
- DEBONO ET AL: "electrophysiological studies of the effects of riluzole on purkinje cells in cerebellar slices" BR J PHARMACOL, vol. 97, 1989, page 584 XP002081721
- DESSI ET AL: "riluzole prevents anoxic injury in cultured cerebellar granule neurons" EUR. J. OF PHARM., vol. 250, no. 2, 1993, pages 325-328, XP002081722

## Description

La présente invention concerne une nouvelle application thérapeutique du 2-amino-6-trifluorométhoxybenzothiazole connu sous la dénomination commune internationale « Riluzole » ou un sel pharmaceutiquement acceptable de ce composé.

Le Riluzole est commercialisé pour le traitement de la sclérose latérale amyotrophique. Ce composé est également utile comme anticonvulsivant, anxiolytique et hypnotique (EP50551), dans le traitement de la schizophrénie (EP305276), dans le traitement des troubles du sommeil et de la dépression (EP305277), dans le traitement des désordres cérébrovasculaires et comme anesthésique (EP282971), dans le traitement des traumatismes spinaux, crâniens ou crânio-spinaux (WO94/13288), comme radiorestaurateur (WO94/15600), dans le traitement de la maladie de Parkinson (WO94/15601), dans le traitement du neuro-sida (WO94/20103), dans le traitement des maladies mitochondriales (WO95/19170).

Le glutamate est l'un des neurotransmetteurs les plus largement répandus et les plus importants du système nerveux. Ses effets sur les neurones sont modulés par des protéines de transport qui font pénétrer le glutamate à l'intérieur des cellules. La structure moléculaire de quatre transporteurs au glutamate est bien connue (Gegelashvili, G. et Schousboe, A., *J. Pharmacol. Exp. Ther*., 52: 6-15, 1997 ; Takahashi, M. et al., *J. Exp. Biol.,* 200: 401-409, 1997) et un cinquième transporteur a récemment été identifié (Arriza, J.L., *et al., Proc. Natl. Acad. Sci. USA*, 94: 4155-4160). Les méthodes de localisation histologique indiquent que ces transporteurs ne sont pas tous présents de manière uniforme dans les différents types de cellules rencontrées dans le système nerveux. Deux des premiers transporteurs identifies. appelés GLAST (ou EAAT1) et GLT-1 (ou EAAT-2), sont localisés de manière prépondérante dans les cellules gliales. Le transporteur EAAC-1 (EAAT-3) est exprimé par les neurones à travers l'ensemble du cerveau. Le transporteur EAAT-4. plus récemment identifié, est exprimé principalement par un type particulier de neurones du cervelet appelé cellules de Purkinje (Nagao, S., *et al., Neurosciences*, 78: 929-933, 1997). Le transporteur le plus récemment identifié est appelé EAAT-5 et est retrouvé au niveau de la rétine de l'oeil.

Les cellules de Purkinje utilisent l'acide gamma-aminobutyrique (GABA) comme neurotransmetteur. Le glutamate étant un précurseur métabolique du GABA (*Biochemistry and the Central Nervous System*, McIlwain, H.M., et Bachelard, H.S. (eds.), Churchill Livingston. Londres, 1971, pp193) l'un des rôles du transporteur EAAT-4 serait de participer à l'approvisionnement en glutamate des cellules de Purkinje du cervelet pour assurer la synthèse de leur neurotransmetteur. Ceci est corroboré par des études récentes qui montrent que la perte du transporteur EAAT-4, contrairement à celle des transporteurs EAAT-1, EAAT-2 et EAAT-3, provoque un dysfonctionnement du cervelet provoquant des symptômes comportementaux à type d'ataxie chez le rongeur (Maragakis, N., *et al*., Soc. Neurosci. Abstr., 23: 1484, 1997).

Il a maintenant été trouvé de manière surprenante que le Riluzole stimule le transport du glutamate dans des préparations de cervelet de rat. Ainsi, le Riluzole peut être utilisé pour la prévention ou le traitement des dysfonctionnements du cervelet. notamment ceux dûs à un mauvais approvisionnement en glutamate des cellules du cervelet. Parmi ces dysfonctionnements on peut citer les ataxies cérébelleuses.

Cet effet est spécifique du cervelet car il ne s'observe pas avec des préparations similaires réalisées à partir d'autres structures du cerveau comme le cortex cérébral et le striatum, ni à partir de moelle épinière. Par ailleurs, l'augmentation par le Riluzole de la capture de glutamate dans le cervelet augmente avec l'âge des rats de la période postnatale à l'âge adulte pour se stabiliser au delà d'environ 10 semaines, c'est-à-dire au dessus d'un poids de 350g.

L'augmentation de la capture de glutamate est évaluée dans une suspension de synaptosomes préparés à partir de cellules de cervelet de rat. Ce type de préparation est connu pour permettre l'étude *in vitro* du transport du glutamate à travers la paroi cellulaire (Kanai *et al., Trends Neurosci*., 16: 365-370, 1993).

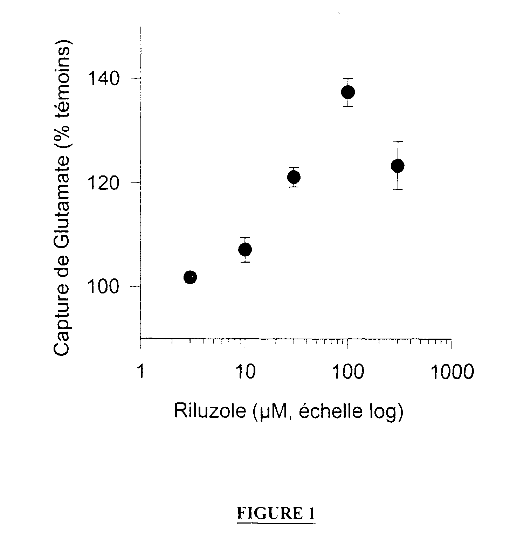

Dans des synaptosomes de cervelet de rat, la capture de glutamate radioactif est augmentée de manière concentration-dépendante par addition de Riluzole à ces préparations (Figure 1).

Les synaptosomes ont été préparés selon une adaptation de la méthode décrite par Robinson, M.B., *et al*. (*Brain Res*., 544: 196-202, 1991). Les tissus de cervelet ou d'autre régions du cerveau de rats mâles de race Sprague-Dawley sont homogénéisés dans une solution de saccharose (0,32M) à 4°C puis centrifugés à 800 g pendant 10 minutes. Le surnageant est ensuite re-centrifugé à 20.000 g pendant 20 minutes. Le culot de centrifugation obtenu est remis en suspension dans une solution de saccharose identique puis centrifugé à nouveau à 20.000 g pendant 20 minutes. Les synaptosomes sont contenus dans ce second culot de centrifugation. Ils sont mis en suspension et utilisés immédiatement pour la mesure de capture de glutamate. Celle-ci est suivie à l'aide de glutamate marqué au tritium ([³H]glutamate) dilué dans une solution de glutamate non-radioactif. Après 3 minutes d'incubation à 37°C, les synaptosomes sont séparés du milieu d'incubation par filtration. L'effet du produit est évalué en comparant la radioactivité retenue par les filtres en absence ou en présence de concentrations croissantes de Riluzole à pH 7,3. Cette radioactivité est proportionnelle à la capture de glutamate. Les résultats sont exprimés en capture spécifique, c'est-à-dire la capture qui dépend de la présence d'ions sodium dans le milieu d'incubation. Elle se distingue de la capture non-spécifique qui correspond à la radioactivité mesurée après remplacement équimolaire du chlorure de sodium du milieu d'incubation par du chlorure de choline. La capture spécifique correspond à la radioactivité totale moins la radioactivité mesurée en présence de chlorure de choline.

Description sommaire de la figure 1 (augmentation par le Riluzole du transport de glutamate dans le cervelet) : le graphe représente la capture de glutamate radiomarqué dans des synaptosomes de cervelet de rat (axe des ordonnées) en fonction de concentrations croissantes de Riluzole (axe des abscisses). Les données sont les moyennes ± ESM de n = 8 observations indépendantes. L'augmentation maximale de capture observée en présence de Riluzole atteint près de 40 % des valeurs témoins. La concentration efficace demi-maximale (CE₅₀) du Riluzole dans cette série expérimentale, calculée par régression sigmoidale, ressort à 24.5 µM.

Comme sels pharmaceutiquement acceptables du Riluzole peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments sont constitués par au moins le Riluzole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale ou rectale.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice. sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau. l'éthanol, le glycerol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les doses dépendent de l'effet recherché, de la durée du traitement et de la voie d'administration utilisée ; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter.

Les exemples suivants illustrent des médicaments selon l'invention :

### Exemple A

On prépare, selon la technique habituelle, des comprimés dosés à 50 mg de produit actif ayant la composition suivante :
- Amino-2 trifluorométhoxy-6 benzothiazole 50 mg
- Mannitol 64 mg
- Cellulose microcristalline 50 mg
- Polyvidone excipient 12 mg
- Carboxyméthylamidon sodique 16 mg
- Talc 4 mg
- Stéarate de magnésium 2 mg
- Silice colloïdale anhydre mg
- Mélange de méthylhydroxypropylcellulose, polyéthylèneglycol 6000, dioxyde de titane (72-3,5-24,5) q.s.p. 1 comprimé pelliculé terminé à 245 mg

### Exemple B

On prépare, selon la technique habituelle, des gélules dosées à 50 mg de produit actif ayant la composition suivante :
- Amino-2 trifluorométhoxy-6 benzothiazole 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### Exemple C

On prépare une solution injectable contenant 10 mg de produit actif avant la composition suivante :
- Amino-2 trifluorométhoxy-6 benzothiazole 10 mg
- Acide benzoïque 80 mg
- Alcool benzylique 0.06 cm3
- Benzoate de sodium 80 mg
- Ethanol à 95 % 0.4 cm3
- Hydroxyde de sodium 24 mg
- Propylène glycol 1.6 cm3
- Eau q.s.p. 4 cm3

L'invention concerne également le procéde de préparation de médicaments utiles dans la prévention ou le traitement des dysfonctionnements du cervelet, notamment ceux dûs à un mauvais approvisionnement en glutamate des cellules du cervelet et, en particulier à la prévention ou le traitement des ataxies cérébelleuses consistant à mélanger le Riluzole ou les sels pharmaceutiquement acceptables de ce composé avec un ou plusieurs diluants et/ou adjuvants compatibles et pharmaceutiquement acceptables.

## Revendications

1. Application du Riluzole ou les sels pharmaceutiquement acceptables de ce composé à la préparation de médicaments pour la prévention ou le traitement des dysfonctionnements du cervelet dûs à un mauvais approvisionnement en glutamate des cellules du cervelet.

2. Application selon la revendication 1 pour la prévention ou le traitement des ataxies cérébelleuses.

3. Application selon l'une des revendications 1 ou 2 pour la préparation d'un médicament comprenant 25 à 200 mg de Riluzole.

## Patentansprüche

1. Verwendung von Riluzol oder den pharmazeutisch akzeptablen Salzen dieser Verbindung zur Herstellung von Arzneimitteln für die Vorbeugung oder Behandlung von Funktionsstörungen des Kleinhirns, die mit einer schlechten Versorgung der Zellen des Kleinhirn mit Glutamat einhergehen.

2. Verwendung nach Anspruch 1 für die Vorbeugung oder Behandlung von cerebellösen Ataxia.

3. Verwendung nach einem der Ansprüche 1 oder 2 zur Herstellung eines Arzneimittels, das 25 mg bis 200 mg Riluzol umfaßt.

## Claims

1. Application of Riluzole or the pharmaceutically acceptable salts of this compound to the preparation of medicaments for the prevention or treatment of cerebellar dysfunction due to a poor supply of glutamate to the cerebellar cells.

2. Application according to Claim 1, for the prevention or treatment of cerebellar ataxia.

3. Application according to either of Claims 1 and 2, for the preparation of a medicament comprising 25 to 200 mg of Riluzole.
